# EUROPEAN PATENT APPLICATION

(11) **EP 2 898 923 A2**
(43) Date of publication of application: **29.07.2015**
(21) Application number: 15158963.7
(22) Date of filing: 26.03.2012
(51) Int. Cl.: A61P 25/28, A61K 31/353, A23L 1/30, A61K 31/70

(54) **Isoflavones for regulating sirtuin gene expression**

(30) Priority: 15.04.2011 US 201161517228 P
(62) Divisional of application: 12770611.7
(71) Applicant: Nestec S.A., 1800 Vevey (CH)
(72) Inventor: Pan, Yuanlong, Chesterfield, MO Missouri 63017 (US); Middleton, Rondo, Paul, Creve Coeur, MO Missouri 63141 (US)
(74) Representative: Rupp, Christian

(57) **Abstract**

The invention provides compositions useful for regulating sirtuin gene expression, mimicking caloric restriction, preventing and treating Alzheimer's disease, increasing longevity and retarding aging in an animal. The compositions comprising one or more isoflavones are to be administered to the animals, preferably in amounts of from about 0.001 to about 10 g/kg/day.

## Description

### Field of the Invention

The invention relates generally to methods for regulating gene expression and particularly to methods for regulating sirtuin gene expression in animals.

### Description of Related Art

Isoflavones are naturally occurring chemical compounds found in plants such as beans and legumes, particularly soy. Isoflavones mimic the effects of estrogen and modulate estrogen metabolism. As a result, isoflavones are known to reduce tumor cell proliferation, induce tumor cell apoptosis, regulate hormone balance, and reduce the risks of breast and prostate cancer, heart disease, osteoporosis, and several other diseases and conditions.

Protein expression is a subcomponent of gene expression. Protein expression denotes the stages after DNA has been translated into polypeptide chains, which are ultimately folded into proteins. Protein expression is commonly used to denote the measurement of the amount or concentration of one or more proteins in a particular cell or tissue.

Sirtuins are a family of NAD+ dependent enzymes that regulate lifespan in lower organisms including yeast, worms and flies. How sirtuins influence lifespan is not fully understood. To date, seven family members have been identified in mammals. SIRT 6 and SIRT7 are nuclear proteins. SIRT3, SIRT4 and SIRT5 are mitochondrial proteins. SIRT1 and SIRT 2 are proteins found both in the nucleus and cytoplasm. Mammalian sirtuins have been connected to cellular stress resistance, genomic stability, tumorgenesis and energy metabolism. *See* Finkel et al. Nature 460, 587-591 (2009*).*

SIRT1 has been shown to mediate lifespan extension associated with caloric restriction. Activation of SIRT1 promotes longevity by regulating energy expenditure during periods of caloric restriction. SIRT1 has also been shown to reduce the production of beta-amyloid plaques. The accumulation of beta-amyloid plaques is associated with Alzheimer's disease. *See* Donmez et al., Cell 142, 320-332 (2010*).* Isoflavone derivatives have demonstrated differential effects on the activation and expression of SIRT1. Resveratrol, an activator of SIRT1, has been reported to promote mitochondrial biogenesis and to protect from metabolic disease. *See* Rasbach et al., J Pharmacol Exp Ther 325, 536-543 (2008*).* While Resveratrol may be useful it is highly sensitive to oxygen and difficult to maintain its stability.

Caloric restriction increases longevity, promotes health, retards aging and aging related diseases by activating sirtuins. Caloric restriction is difficult to practice in humans and pets.

There is, therefore, a need for compositions and methods useful for regulating sirtuin gene expression in an animal. There is also a need for compositions and methods for regulating sirtuin gene expression and thereby regulating sirtuin expression, mimicking caloric restriction, preventing and treating Alzheimer's disease, increasing longevity, and retarding aging. Such therapies would be particularly useful in aging humans and other animals to improve the overall quality of life for all involved. For companion animals, these therapies would lead to improved owner satisfaction and would improve the owner-companion animal bond.

### SUMMARY OF THE INVENTION

It is, therefore, an object of the invention to provide compositions useful for regulating sirtuin gene expression, mimicking caloric restriction, preventing and treating Alzheimer's disease, increasing longevity and retarding aging in an animal.

Other and further objects, features, and advantages of the present invention will be readily apparent to those skilled in the art.

The invention is directed to a composition comprising one or more isoflavones in a therapeutipically effective amount for use in one or more of the treatment or prevention of Alzheimer's Disease, the mimicking of caloric restriction, increasing longevity, or in retarding aging in animals, wherein said composition comprises the isoflavone equol or a precursor of equol said precursor optionally being daidzein, daidzin, or formononetin. Said isoflavones can regulate sirtuin gene expression in an animal. The sirtuin gene expressed can be at least one of SIRT1, SIRT2, SIRT3, SIRT4, SIRT5 or SIRT7. The sirtuin gene expressed can be at least one of SIRT2 or SIRT3. The sirtuin gene expressed can be at least one of SIRT4, SIRT5 or SIRT7. The composition can further comprise an isoflavone selected from the group consisting of isoflavones in the form of aglycons, glucosides, acetylglucosides, and malonylglucosides. The composition can further comprise an isoflavone selected from the group consisting of biochanin A, daidzein, daidzin, glycitein, formononetin, genistein, irilone, luteone, prunetin, pratensein, and glycitinn. The isoflavones can be administered in amounts of from about 0.001 to about 10 g/kg/day. The isoflavones can be administered to the animal on a regular basis. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics, inactivated probiotics, and components of inactivated probiotics that promote health benefits similar to or the same as the probiotics.

The animal can be a human or a companion animal, said companion animal optionally being a canine or a feline. The animal can be an aging animal.

### DETAILED DESCRIPTION OF THE INVENTION

### Definitions

The term "isoflavones" means isoflavones and their natural or synthetic analogs, derivatives, precursors, and metabolites useful in the invention. Isoflavones refers to 3-phenylchromones, isomeric forms of flavones in which the benzene group is attached to the 3 position of the benzopyran ring instead of the 2 position. Isoflavones may be found in a number of sources, including, but not limited to, soy. Non-limiting examples of isoflavones include daidzein; daidzin; 6-O-malonyl daidzein; 6-O-acetyl daidzein; genistein; 6-O-malonyl genistein; 6-O-acetyl genistein; glycitein; 6-O-malonyl glycitein; 6-O-acetyl glycitein; Biochanin A; formononetin; irilone; prunetin; pratensein; glycitinn; dihydrodaidzein; equol; O-desmethylangolensin; daidzein 7,4'-di-O-sulfate; daidzein 7-O-beta-D-glucuronide; daidzein 4'-O-sulfate; 6,7,5'-trihydroxyisoflavone; 6,7,3',4'-tetrahydroxyisoflavone; 7,8,4'-trihydroxyisoflavone; 5,6,7,4'-tetrahydroxyisoflavone; dihydrogenistein; *p*-ethylphenol; 3',4',5, 7-tetrahydroxyisoflavone; genistein 4'-O-sulfate; genistein 7-O-beta-D-glucuronide; genistein 4'-O-sulfate; and 4',5,7-trihydroxyisoflavanone.

The term "animal" means any animal that can benefit from the regulation of sirtuin gene expression, e.g., a human, avian, bovine, canine, equine, feline, hicrine, lupine, murine, ovine, and porcine animals.

The term "companion animal" means domesticated animals such as cats, dogs, rabbits, guinea pigs, ferrets, hamsters, mice, gerbils, horses, cows, goats, sheep, donkeys, pigs, and the like.

The term "therapeutically-effective amount" means an amount of a compound of the present invention that (i) treats or prevents the particular disease, condition, or disorder, (ii) attenuates, ameliorates, or eliminates one or more symptoms of the particular disease, condition, or disorder, or (iii) prevents or delays the onset of one or more symptoms of the particular disease, condition, or disorder described herein.

The terms "treating", "treat", and "treatment" embrace both preventative, *i.e.,* prophylactic, and palliative treatment.

The terms "pharmaceutically acceptable" and "nutraceutically acceptable" indicates that the substance or composition must be compatible chemically and/or toxicologically, with the other ingredients comprising a formulation, and/or the mammal being treated therewith.

The term "health and/or wellness of an animal" means the complete physical, mental, and social well being of the animal, not merely the absence of disease or infirmity.

The term "extending the prime" means extending the number of years an animal lives a healthy life and not just extending the number of years an animal lives, *e.g.,* an animal would be healthy in the prime of its life for a relatively longer time.

The term "in conjunction" means that compositions of the invention are administered to an animal (1) together in a food composition or (2) separately at the same or different frequency using the same or different administration routes at about the same time or periodically. "Periodically" means that compositions are administered on a schedule acceptable for specific compounds or compositions. "About the same time" generally means that compositions are administered at the same time or within about 72 hours of each other.

The term "dietary supplement" means a product that is intended to be ingested in addition to a normal animal diet. Dietary supplements may be in any form, e.g., solid, liquid, gel, tablet, capsule, powder, and the like. Preferably they are provided in convenient dosage forms, *e.g.,* in sachets. Dietary supplements can be provided in bulk consumer packages such as bulk powders, liquids, gels, or oils. Similarly such supplements can be provided in bulk quantities to be included in other food items such as snacks, treats, supplement bars, beverages, and the like.

The term "aging" means being of an advanced age such that an animal has reached or exceeded 50% of the average life expectancy for the animal's species and/or breed within such species. For example, if the average life expectancy for a given breed of dog is 12 years, then an "aging animal" within that breed is 6 years old or older.

The term "food" or "food product" or "food composition" means a product or composition that is intended for ingestion by an animal, including a human, and provides nutrition to the animal.

The term "regular basis" means at least monthly dosing with compositions of the present invention and more preferably weekly dosing. More frequent dosing or consumption, such as twice or three times weekly, is preferred in certain embodiments. Still more preferred are regimens that comprise at least once daily consumption, e.g., when compositions of the present invention are a component of a food composition that is consumed at least once daily.

The term "single package" means that the components of a kit are physically associated in or with one or more containers and considered a unit for manufacture, distribution, sale, or use. Containers include, but are not limited to, bags, boxes, cartons, bottles, packages such as shrink wrap packages, stapled or otherwise affixed components, or combinations thereof. A single package may be containers of individual compositions of the present invention and food compositions physically associated such that they are considered a unit for manufacture, distribution, sale, or use.

The term "virtual package" means that the components of a kit are associated by directions on one or more physical or virtual kit components instructing the user how to obtain the other components, *e.g.,* in a bag or other container containing one component and directions instructing the user to go to a website, contact a recorded message or a fax-back service, view a visual message, or contact a caregiver or instructor to obtain instructions on how to use the kit or safety or technical information about one or more components of a kit.

The dosages expressed herein are in milligrams per kilogram of body weight per day (mg/kg/day) unless expressed otherwise.

All percentages expressed herein are by weight of the composition on a dry matter basis unless specifically stated otherwise. The skilled artisan will appreciate that the term "dry matter basis" means that an ingredient's concentration or percentage in a composition is measured or determined after any free moisture in the composition has been removed.

As used herein, ranges are used herein in shorthand, so as to avoid having to list and describe each and every value within the range. Any appropriate value within the range can be selected, where appropriate, as the upper value, lower value, or the terminus of the range.

As used herein, the singular form of a word includes the plural, and vice versa, unless the context clearly dictates otherwise. Thus, the references "a", "an", and "the" are generally inclusive of the plurals of the respective terms. For example, reference to "a supplement", "a method", or "a food" includes a plurality of such "supplements", "methods", or "foods." Similarly, the words "comprise", "comprises", and "comprising" are to be interpreted inclusively rather than exclusively. Likewise the terms "include", "including" and "or" should all be construed to be inclusive, unless such a construction is clearly prohibited from the context. Similarly, the term "examples," particularly when followed by a listing of terms, is merely exemplary and illustrative and should not be deemed to be exclusive or comprehensive.

The methods and compositions and other advances disclosed here are not limited to particular methodology, protocols, and reagents described herein because, as the skilled artisan will appreciate, they may vary. Further, the terminology used herein is for the purpose of describing particular embodiments only, and is not intended to, and does not, limit the scope of that which is disclosed or claimed.

Unless defined otherwise, all technical and scientific terms, terms of art, and acronyms used herein have the meanings commonly understood by one of ordinary skill in the art in the field(s) of the invention, or in the field(s) where the term is used. Although any compositions, methods, articles of manufacture, or other means or materials similar or equivalent to those described herein can be used in the practice of the present invention, the preferred compositions, methods, articles of manufacture, or other means or materials are described herein.

### Detailed description

Disclosed is a method for regulating sirtuin gene expression in an animal comprising administering one or more isoflavones to the animal in a therapeutically effective amount. The sirtuin gene expressed can be at least one of SIRT1, SIRT2, SIRT3, SIRT4, SIRT5 or SIRT7. The sirtuin gene expressed can be at least one of SIRT2 or SIRT3. The sirtuin gene expressed can be at least one of SIRT4, SIRT5 or SIRT7. The isoflavones can be selected from the group consisting of isoflavones in the form of aglycons, glucosides, acetylglucosides, and malonylglucosides. The isoflavones can be selected from the group consisting of biochanin A, daidzein, daidzin, glycitein, formononetin, equol, genistein, irilone, luteone, prunetin, pratensein, and glycitinn. The isoflavones can be administered in amounts of from about 0.001 to about 10 g/kg/day. The isoflavones can be administered to the animal on a regular basis. The soflavones can be administered in amounts of from about 0.1 to about 10 g. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics; inactivated probiotics; components of inactivated probiotics that promote health benefits similar to or the same as the probiotics; and one or more prebiotics. The animal can be a human or a companion animal. The companion animal can be a canine. The companion animal can be a feline. The animal can be an aging animal.

Disclosed is a method for mimicking caloric restriction in an animal comprising administering one or more isoflavones to the animal in a therapeutically effective amount for regulating sirtuin gene expression and thereby mimicking caloric restriction. The sirtuin gene expressed can be at least one of SIRT1, SIRT2, SIRT3, SIRT4, SIRT5 or SIRT7. The sirtuin gene expressed can be at least one of SIRT2 or SIRT3. The sirtuin gene expressed can be at least one of SIRT4, SIRT5 or SIRT7. The isoflavones can be selected from the group consisting of isoflavones in the form of aglycons, glucosides, acetylglucosides, and malonylglucosides. The isoflavones can be selected from the group consisting of biochanin A, daidzein, daidzin, glycitein, formononetin, equol, genistein, irilone, luteone, prunetin, pratensein, and glycitinn. The isoflavones can be administered in amounts of from about 0.001 to about 10 g/kg/day. The isoflavones can be administered to the animal on a regular basis. The soflavones can be administered in amounts of from about 0.1 to about 10 g. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics; inactivated probiotics; components of inactivated probiotics that promote health benefits similar to or the same as the probiotics; and one or more prebiotics. The animal can be a human or a companion animal. The companion animal can be a canine. The companion animal can be a feline. The animal can be an aging animal.

Disclosed is a method for preventing and treating Alzheimer's disease in an animal comprising administering one or more isoflavones to the animal in a therapeutically effective amount for regulating sirtuin gene expression and thereby preventing and treating Alzheimer's disease. The sirtuin gene expressed can be at least one of SIRT1, SIRT2, SIRT3, SIRT4, SIRT5 or SIRT7. The sirtuin gene expressed can be at least one of SIRT2 or SIRT3. The sirtuin gene expressed can be at least one of SIRT4, SIRT5 or SIRT7. The isoflavones can be selected from the group consisting of isoflavones in the form of aglycons, glucosides, acetylglucosides, and malonylglucosides. The isoflavones can be selected from the group consisting of biochanin A, daidzein, daidzin, glycitein, formononetin, equol, genistein, irilone, luteone, prunetin, pratensein, and glycitinn. The isoflavones can be administered in amounts of from about 0.001 to about 10 g/kg/day. The isoflavones can be administered to the animal on a regular basis. The soflavones can be administered in amounts of from about 0.1 to about 10 g. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics; inactivated probiotics; components of inactivated probiotics that promote health benefits similar to or the same as the probiotics; and one or more prebiotics. The animal can be a human or a companion animal. The companion animal can be a canine. The companion animal can be a feline. The animal can be an aging animal.

Disclosed is a method for increasing longevity in an animal comprising administering one or more isoflavones to the animal in a therapeutically effective amount for regulating sirtuin gene expression and thereby increasing longevity. The sirtuin gene expressed can be at least one of SIRT1, SIRT2, SIRT3, SIRT4, SIRT5 or SIRT7. The sirtuin gene expressed can be at least one of SIRT2 or SIRT3. The sirtuin gene expressed can be at least one of SIRT4, SIRT5 or SIRT7. The isoflavones can be selected from the group consisting of isoflavones in the form of aglycons, glucosides, acetylglucosides, and malonylglucosides. The isoflavones can be selected from the group consisting of biochanin A, daidzein, daidzin, glycitein, formononetin, equol, genistein, irilone, luteone, prunetin, pratensein, and glycitinn. The isoflavones can be administered in amounts of from about 0.001 to about 10 g/kg/day. The isoflavones can be administered to the animal on a regular basis. The soflavones can be administered in amounts of from about 0.1 to about 10 g. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics; inactivated probiotics; components of inactivated probiotics that promote health benefits similar to or the same as the probiotics; and one or more prebiotics. The animal can be a human or a companion animal. The companion animal can be a canine. The companion animal can be a feline. The animal can be an aging animal.

Disclosed is a method for retarding aging in an animal comprising administering one or more isoflavones to the animal in a therapeutically effective amount for regulating sirtuin gene expression and thereby retarding aging in animals. The sirtuin gene expressed can be at least one of SIRT1, SIRT2, SIRT3, SIRT4, SIRT5 or SIRT7. The sirtuin gene expressed can be at least one of SIRT2 or SIRT3. The sirtuin gene expressed can be at least one of SIRT4, SIRT5 or SIRT7. The isoflavones can be selected from the group consisting of isoflavones in the form of aglycons, glucosides, acetylglucosides, and malonylglucosides. The isoflavones can be selected from the group consisting of biochanin A, daidzein, daidzin, glycitein, formononetin, equol, genistein, irilone, luteone, prunetin, pratensein, and glycitinn. The isoflavones can be administered in amounts of from about 0.001 to about 10 g/kg/day. The isoflavones can be administered to the animal on a regular basis. The soflavones can be administered in amounts of from about 0.1 to about 10 g. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics; inactivated probiotics; components of inactivated probiotics that promote health benefits similar to or the same as the probiotics; and one or more prebiotics. The animal can be a human or a companion animal. The companion animal can be a canine. The companion animal can be a feline. The animal can be an aging animal.

Disclosed is a composition comprising isoflavones in a therapeutically effective amount for one or more of regulating sirtuin gene expression, mimicking caloric restriction, preventing and treating Alzheimer's disease, increasing longevity and retarding aging in animals. The composition can comprise isoflavones in a therapeutically effective amount for regulating sirtuin gene expression and thereby capable of one or more of mimicking caloric restriction, preventing and treating Alzheimer's disease, increasing longevity and retarding aging in animals. The composition can contain isoflavones in amounts sufficient to administer isoflavones to an animal in amounts to about from about 0.001 to about 10 g/kg/day.

Disclosed is a pharmaceutical or nutraceutical composition comprising isoflavones and one or more pharmaceutically or nutraceutically acceptable carrier, diluents or excipients.

Disclosed is a package comprising at least one material suitable for containing isoflavones and a label affixed to the package containing a word or words, picture, design, acronym, slogan, phrase, or other device, or combination thereof that indicates that the contents of the package contains isoflavones.

91. Disclosed is that the label affixed to the package contains a word or words, picture, design, acronym, slogan, phrase, or other device, or combination thereof that indicates that the contents of the package contains isoflavones with beneficial properties relating to promoting healthy aging.

Disclosed is that the beneficial properties are one or more of regulating sirtuin gene expression, mimicking caloric restriction, preventing and treating Alzheimer's disease, increasing longevity, retarding aging, and promoting healthy aging. The package can further comprise isoflavones. The package can further comprise at least one window.

Disclosed is a means for communicating information about, or instructions for, one or more of (1) using isoflavones for regulating sirtuin gene expression; (2) using isoflavones for mimicking caloric restriction; (3) using isoflavones for preventing and treating Alzheimer's disease; (4) using isoflavones for increasing longevity; (5) using isoflavones for retarding aging; (6) using isoflavones for promoting healthy aging; (7) contact information for consumers to use if they have a question regarding the methods and compositions of the invention; and (8) nutritional information about isoflavones; the means comprising one or more of a physical or electronic document, digital storage media, optical storage media, audio presentation, audiovisual display, or visual display containing the information or instructions. The means can be selected from the group consisting of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof.

Disclosed is a kit suitable for administering isoflavones to an animal comprising in separate containers in a single package or in separate containers in a virtual package, as appropriate for the kit component, isoflavones and one or more of (1) one or more ingredients suitable for consumption by an animal; (2) instructions for how to combine isoflavones and other kit components to produce a composition useful for regulating sirtuin gene expression, mimicking caloric restriction, preventing and treating Alzheimer's disease, increasing longevity, and retarding aging; (3) instructions for how to use isoflavones for regulating sirtuin gene expression; (4) instructions for how to use isoflavones for mimicking caloric restriction; (5) instructions for how to use isoflavones for preventing and treating Alzheimer's disease; (6) instructions for how to use isoflavones for increasing longevity; (7) instructions for how to use isoflavones for retarding aging; (8) one or more probiotics; (9) one or more inactivated probiotics; (10) one or more components of inactivated probiotics that promote health benefits similar to or the same as the probiotics; (11) one or more prebiotics; (12) a device for preparing or combining the kit components to produce a composition suitable for administration to an animal; and (13) a device for administering the combined or prepared kit components to an animal. The isoflavones can be in a sachet. The kit can comprise isoflavones and one or more ingredients suitable for consumption by an animal. The kit can comprise isoflavones and one or more probiotics.

Disclosed is a method for regulating SIRT1, SIRT2, SIRT3, SIRT4, SIRT5, SIRT6, or SIRT7 expression in an animal comprising administering one or more isoflavones to the animal in a therapeutically effective amount.

Disclosed is a method for regulating sirtuin expression in an animal comprising administering one or more isoflavones to the animal in a therapeutically effective amount.The isoflavones can be selected from the group consisting of isoflavones in the form of aglycons, glucosides, acetylglucosides, and malonylglucosides. The isoflavones can be selected from the group consisting of biochanin A, daidzein, daidzin, glycitein, formononetin, equol, genistein, irilone, luteone, prunetin, pratensein, and glycitinn. The isoflavones can be administered in amounts of from about 0.001 to about 10 g/kg/day. The isoflavones can be administered to the animal on a regular basis. The soflavones can be administered in amounts of from about 0.1 to about 10 g. The isoflavones can be administered to the animal as a dietary supplement or a food composition. The isoflavones can be administered in a food composition and the isoflavones can comprise from about 0.001 to about 40% of the food composition. The food composition can be formulated to provide complete and balanced nutrition for the animal. The food composition or dietary supplement can further comprise one or more probiotics; inactivated probiotics; components of inactivated probiotics that promote health benefits similar to or the same as the probiotics; and one or more prebiotics. The animal can be a human or a companion animal. The companion animal can be a canine. The companion animal can be a feline. The animal can be an aging animal.

In one aspect, the invention provides methods for regulating sirtuin gene expression in animals. The methods comprise administering one or more isoflavones to the animals in a therapeutically effective amount for regulating sirtuin gene expression.

In another aspect, the invention provides methods for mimicking caloric restriction in animals. The methods comprise administering one or more isoflavones to the animals in a therapeutically effective amount for regulating sirtuin gene expression and thereby mimicking caloric restriction in animals.

In another aspect, the invention provides methods for preventing and treating Alzheimer's disease in animals. The methods comprise administering one or more isoflavones to the animals in a therapeutically effective amount for regulating sirtuin gene expression and thereby preventing and treating Alzheimer's disease in animals.

In another aspect, the invention provides methods for increasing longevity for animals. The methods comprise administering one or more isoflavones to the animals in a therapeutically effective amount for regulating sirtuin gene expression and thereby increasing longevity for the animals.

In another aspect, the invention provides methods for retarding aging in animals. The methods comprise administering one or more isoflavones to the animals in a therapeutically effective amount for regulating sirtuin gene expression and thereby retarding aging in animals.

The inventions are based upon the discovery that administering isoflavones to animals regulates sirtuin gene expression in the animals and the discovery that sirtuins affect various functions relating to the animal, e.g., mimicking caloric restriction, preventing and treating Alzheimer's disease, increasing longevity, retarding aging, and the like for animals.

Regulating sirtuin gene expression regulates the amount of serotonins in the animal. In preferred embodiments, the siritunin genes are upregulated and therefore increase the amount of sirtuins in animals.

The sirtuin genes and serotonins regulated are SIRT2 and SIRT3. Data developed to support the invention show that the gene for SIRT6 is not regulated by administering isoflavones to the animals.

In one embodiment, the sirtuin gene expressed is at least one of SIRT1, SIRT2, SIRT3, SIRT4, SIRT5 or SIRT7. In another embodiment, the sirtuin gene expressed is at least one of SIRT2 or SIRT3. In another embodiment, the sirtuin gene expressed is at least one of SIRT4, SIRT5 or SIRT7.

In another aspect, the invention provides a method for regulating SIRT1 expression in an animal comprising administering one or more isoflavones to the animal in a therapeutically effective amount.

In another aspect, the invention provides a method for regulating SIRT2 expression in an animal comprising administering one or more isoflavones to the animal in a therapeutically effective amount.

In another aspect, the invention provides a method for regulating SIRT3 expression in an animal comprising administering one or more isoflavones to the animal in a therapeutically effective amount.

In another aspect, the invention provides a method for regulating SIRT4 expression in an animal comprising administering one or more isoflavones to the animal in a therapeutically effective amount.

In another aspect, the invention provides a method for regulating SIRT5 expression in an animal comprising administering one or more isoflavones to the animal in a therapeutically effective amount.

In another aspect, the invention provides a method for regulating SIRT7 expression in an animal comprising administering one or more isoflavones to the animal in a therapeutically effective amount.

In various embodiments, the animal is any animal that has a need for regulating sirtuin gene expression, including but not limited to, mimicking caloric restriction, preventing and treating Alzheimer's disease, increasing longevity and retarding aging. In one embodiment, the animal is a human or companion animal, preferably a canine or a feline. In another embodiment, the animal is an aging animal.

The isoflavones are any isoflavones known to skilled artisans. In various embodiments, the isoflavones are selected from the group consisting of isoflavones in the form of aglycons, glucosides, acetylglucosides, and malonylglucosides. Preferably the isoflavones are selected from the group consisting of biochanin A, daidzein, daidzin, glycitein, formononetin, equol, genistein, irilone, luteone, prunetin, pratensein, and glycitinn. In one embodiment, the isoflavones are soy isoflavones obtained from soy or administered to the animal by feeding soy or soy extracts to the animal. In another embodiment, the isoflavones are isoflavones substituted with one or more lignans or coumestans such as pinoresinol, lariciresinol, secoisolariciresinol, matairesinol, hydroxymatairesinol, syringaresinol, sesamin, enterodiol, enterolactone, and coumestrol.

Any amount of isoflavone that regulates sirtuin gene expression is administered to the animals.

In preferred embodiments, one or more isoflavones are administered to the animals in amounts of from about 0.1 to about 10 grams of isoflavones, preferably from about 0.5 to about 7.5 grams, more preferably from about 1 to about 5 grams.

In various embodiments, the isoflavones are administered to the animals in amounts of from about 0.1 to about 10 grams of isoflavones per day (g/day), preferably from about 0.5 to about 7.5 g/day, more preferably from about 1 to about 5 g/day.

In other embodiments, the isoflavones are administered to the animals in amounts of from about 0.001 to about 10 grams of isoflavones per kilogram of body weight (g/kg/bw), preferably from about 0.05 to about 5 g/kg/bw, more preferably from about 0.01 to about 1 g/kg/bw.

In further embodiments, the isoflavones are administered to the animals in amounts of from about 0.001 to about 10 grams of isoflavones per kilogram of body weight per day (g/kg/bw/day), preferably from about 0.05 to about 5 g/kg/bw/day, more preferably from about 0.01 to about 1 g/kg/bw/day.

In another aspect, the present invention provides compositions comprising isoflavones in a therapeutically effective amount for regulating sirtuin gene expression. In another embodiment, the present invention provides compositions comprising isoflavones in a therapeutically effective amount for regulating sirtuin gene expression and thereby capable of one or more of mimicking caloric restriction, preventing and treating Alzheimer's disease, increasing longevity and retarding aging in animals.

Compositions of the present invention can be administered to the animal in any suitable form using any suitable administration route. For example, the compositions can be administered in a food composition, in a dietary supplement, in a pharmaceutical composition, in a nutraceutical composition, or as a medicament. Similarly, the compositions can be administered using a variety of administration routes, including oral, intranasal, intravenous, intramuscular, intragastric, transpyloric, subcutaneous, rectal, and the like. Preferably, the compositions are administered to an animal orally. Most preferably, the compositions are administered orally to an animal as a dietary supplement or as an ingredient in a food composition.

In a preferred embodiment, the compositions of the present invention are administered to an animal as an ingredient in a food composition suitable for consumption by an animal, including humans and companion animals such as dogs and cats. Such food compositions include complete foods intended to supply the necessary dietary requirements for an animal or food supplements such as animal treats.

In various embodiments, food compositions such as pet food compositions or pet treat compositions comprise from about 5% to about 50% crude protein. The crude protein material may comprise vegetable proteins such as soybean meal, soy protein concentrate, corn gluten meal, wheat gluten, cottonseed, and peanut meal, or animal proteins such as casein, albumin, and meat protein. Examples of meat protein useful herein include beef, pork, lamb, equine, poultry, fish, and mixtures thereof.

The food compositions may further comprise from about 5% to about 40% fat. Examples of suitable fats include animal fats and vegetable fats. Preferably the fat source is an animal fat source such as tallow or grease. Vegetable oils such as corn oil, sunflower oil, safflower oil, rape seed oil, soy bean oil, olive oil and other oils rich in monounsaturated and polyunsaturated fatty acids, may also be used.

The food compositions may further comprise from about 10% to about 60% carbohydrate. Examples of suitable carbohydrates include grains or cereals such as rice, corn, millet, sorghum, alfalfa, barley, soybeans, canola, oats, wheat, rye, triticale and mixtures thereof. The compositions may also optionally comprise other materials such as dried whey and other dairy by-products.

The moisture content for such food compositions varies depending on the nature of the food composition. The food compositions may be dry compositions (e.g., kibble), semi-moist compositions, wet compositions, or any mixture thereof. In a preferred embodiment, the composition is a complete and nutritionally balanced pet food. In this embodiment, the pet food may be a "wet food", "dry food", or food of "intermediate moisture" content. "Wet food" describes pet food that is typically sold in cans or foil bags and has a moisture content typically in the range of about 70% to about 90%. "Dry food" describes pet food that is of a similar composition to wet food but contains a limited moisture content typically in the range of about 5% to about 15% or 20% (typically in the form or small biscuit-like kibbles). In one preferred embodiment, the compositions have moisture content from about 5% to about 20%. Dry food products include a variety of foods of various moisture contents, such that they are relatively shelf-stable and resistant to microbial or fungal deterioration or contamination. Also preferred are dry food compositions that are extruded food products such as pet foods or snack foods for either humans or companion animals.

The food compositions may also comprise one or more fiber sources. The term "fiber" includes all sources of "bulk" in the food whether digestible or indigestible, soluble or insoluble, fermentable or nonfermentable. Preferred fibers are from plant sources such as marine plants but microbial sources of fiber may also be used. A variety of soluble or insoluble fibers may be utilized, as will be known to those of ordinary skill in the art. The fiber source can be beet pulp (from sugar beet), gum arabic, gum talha, psyllium, rice bran, carob bean gum, citrus pulp, pectin, fructooligosaccharide, short chain oligofructose, mannanoligofructose, soy fiber, arabinogalactan, galactooligosaccharide, arabinoxylan, or mixtures thereof.

Alternatively, the fiber source can be a fermentable fiber. Fermentable fiber has previously been described to provide a benefit to the immune system of a companion animal. Fermentable fiber or other compositions known to skilled artisans that provide a prebiotic to enhance the growth of probiotics within the intestine may also be incorporated into the composition to aid in the enhancement of the benefit provided by the present invention to the immune system of an animal.

In some embodiments, the ash content of the food composition ranges from less than 1% to about 15%, preferably from about 5% to about 10%.

In a preferred embodiment, the composition is a food composition comprising and from about 15% to about 50% protein, from about 5% to about 40% fat, from about 5% to about 10% ash content, and having a moisture content of about 5% to about 20%. In other embodiments, the food composition further comprises prebiotics or probiotics as described herein.

When administered in a food composition, the amount of isoflavones as a percentage of the composition is from about 0.1 to about 40% of the food composition, preferably from about 3 to about 30%, more preferably from about 5 to about 20%. In various embodiments, food compositions comprise about 1%, 2%, 4%, 6%, 8%, 10%, 12%, 14%, 16%, 18%, 20%, 22%, 24%, 26%, 28%, 30%, 32%, 34%, 36%, 38%, or 40%.

In another embodiment, the compositions are administered to an animal in a dietary supplement. The dietary supplement can have any suitable form such as a gravy, drinking water, beverage, yogurt, powder, granule, paste, suspension, chew, morsel, treat, snack, pellet, pill, capsule, tablet, sachet, or any other suitable delivery form. The dietary supplement can comprise the compositions and optional compounds such as vitamins, preservatives, probiotics, prebiotics, and antioxidants. This permits the supplement to be administered to the animal in small amounts, or in the alternative, can be diluted before administration to an animal. The dietary supplement may require admixing with a food composition or with water or other diluent prior to administration to the animal. When administered in a dietary supplement, the compositions comprise from about 0.1 to about 90% of the supplement, preferably from about 3 to about 70%, more preferably from about 5 to about 60%.

In another embodiment, the compositions are administered to an animal in a pharmaceutical or nutraceutical composition. The pharmaceutical composition comprises the compositions of the present invention and one or more pharmaceutically or nutraceutically acceptable carriers, diluents, or excipients. Generally, pharmaceutical compositions are prepared by admixing a compound or composition with excipients, buffers, binders, plasticizers, colorants, diluents, compressing agents, lubricants, flavorants, moistening agents, and the like, including other ingredients known to skilled artisans to be useful for producing pharmaceuticals and formulating compositions that are suitable for administration to an animal as pharmaceuticals. When administered in a pharmaceutical or nutraceutical composition, the compositions comprise from about 0.1 to about 90% of the composition of the present invention, preferably from about 3 to about 70%, more preferably from about 5 to about 60%.

The compositions of the present invention can be administered to the animal on an as-needed, on an as-desired basis, or on a regular basis. A goal of administration on a regular basis is to provide the animal with a regular and consistent dose of the compositions or the direct or indirect metabolites that result from such ingestion. Such regular and consistent dosing will tend to create constant blood levels of the compositions and their direct or indirect metabolites. Thus, administration on a regular basis can be once monthly, once weekly, once daily, or more than once daily. Similarly, administration can be every other day, week, or month, every third day, week, or month, every fourth day, week, or month, and the like. Administration can be multiple times per day. When utilized as a supplement to ordinary dietetic requirements, the compositions may be administered directly to the animal, e.g., orally or otherwise. The compositions can alternatively be contacted with, or admixed with, daily feed or food, including a fluid, such as drinking water, or an intravenous connection for an animal that is receiving such treatment. Administration can also be carried out as part of a dietary regimen for an animal. For example, a dietary regimen may comprise causing the regular ingestion by the animal of the compositions in an amount effective to accomplish the methods of the present invention.

According to the methods of the invention, administration of the compositions, including administration as part of a dietary regimen, can span a period ranging from parturition through the adult life of the animal. In various embodiments, the animal is a human or companion animal such as a dog or cat. In certain embodiments, the animal is a young or growing animal. In more preferred embodiments, the animal is an aging animal. In other embodiments administration begins, for example, on a regular or extended regular basis, when the animal has reached more than about 30%, 40%, or 50% of its projected or anticipated lifespan. In some embodiments, the animal has attained 40, 45, or 50% of its anticipated lifespan. In yet other embodiments, the animal is older having reached 60, 66, 70, 75, or 80% of its likely lifespan. A determination of lifespan may be based on actuarial tables, calculations, estimates, or the like, and may consider past, present, and future influences or factors that are known to positively or negatively affect lifespan. Consideration of species, gender, size, genetic factors, environmental factors and stressors, present and past health status, past and present nutritional status, stressors, and the like may also influence or be taken into consideration when determining lifespan.

The compositions of the present invention are administered to an animal for a time required to accomplish one or more objectives of the invention, e.g., regulating sirtuin gene expression; mimicking caloric restriction; preventing and treating Alzheimer's disease; increasing longevity; retarding aging in animals; improving the quality of life; and promoting the health and wellness in an animal. Preferably, the compositions are administered to an animal on a regular basis.

In another aspect, the invention provides therapeutic compositions comprising the compositions of the present invention in a therapeutically effective amount for one or more of regulating sirtuin gene expression; mimicking caloric restriction; preventing and treating Alzheimer's disease, increasing longevity; retarding aging in animals; improving the quality of life; and promoting the health and wellness in an animal. The therapeutic compositions contain the compositions of the present invention in amounts sufficient to administer the compositions of the present invention to an animal in amounts of from about 0.005 to about 1000 mg/kg/day, preferably from about 0.01 to about 500 mg/kg/day, most preferably from about 0.05 to about 250 mg/kg/day when the compositions are administered as anticipated or recommended for a particular composition. Typically, the compositions of the present invention comprise from about 1 to about 90% of a therapeutic composition, preferably from about 3 to about 70%, more preferably from about 5 to about 60%. In certain embodiments, the compositions of the present invention comprise over 90% of a therapeutic composition.

In various embodiments, the compositions further comprise one or more substances such as vitamins, minerals, probiotics, prebiotics, salts, and functional additives such as palatants, colorants, emulsifiers, and antimicrobial or other preservatives. Minerals that may be useful in such compositions include, for example, calcium, phosphorous, potassium, sodium, iron, chloride, boron, copper, zinc, magnesium, manganese, iodine, selenium, and the like. Examples of additional vitamins useful herein include such fat soluble vitamins as A, D, E, and K. Inulin, amino acids, enzymes, coenzymes, and the like may be useful to include in various embodiments.

In various embodiments, the compositions contain at least one of (1) one or more probiotics; (2) one or more inactivated probiotics; (3) one or more components of inactivated probiotics that promote health benefits similar to or the same as the probiotics, e.g., proteins, lipids, glycoproteins, and the like; (4) one or more prebiotics; and (5) combinations thereof. The probiotics or their components can be integrated into the compositions comprising the compositions (e.g., uniformly or non-uniformly distributed in the compositions) or applied to the compositions comprising the compositions (e.g., topically applied with or without a carrier). Such methods are known to skilled artisans, e.g., US5968569 and related patents.

Typical probiotics include, but are not limited to, probiotic strains selected from *Lactobacilli, Bifidobacteria,* or *Enterococci, e.g., Lactobacillus reuteii, Lactobacillus acidophilus, Lactobacillus animalis, Lactobacillus ruminis, Lactobacillus johnsonii, Lactobacillus casei, Lactobacillus paracasei, Lactobacillus rhamnosus*, *Lactobacillus fermentum,* and *Bifidobacterium sp., Enterococcus faecium* and *Enterococcus sp.* In some embodiments, the probiotic strain is selected from the group consisting of *Lactobacillus reuteri* (NCC2581; CNCM I-2448), *Lactobacillus reuteri* (NCC2592; CNCM I-2450), *Lactobacillus rhamnosus* (NCC2583; CNCM I-2449), *Lactobacillus reuteri* (NCC2603; CNCM 1-2451), *Lactobacillus reuteri* (NCC2613; CNCM I-2452), *Lactobacillus acidophilus* (NCC2628; CNCM I-2453), *Bifidobacterium adolescentis* (*e.g.,* NCC2627), *Bifidobacterium sp.* NCC2657 or *Enterococcus faecium* SF68 (NCIMB 10415). The compositions comprising the compositions of the present invention contain probiotics in amounts sufficient to supply from about 10⁴ to about 10¹² cfu/animal/day, preferably from 10⁵ to about 10¹¹ cfu/animal/day, most preferably from 10⁷ to 10¹⁰ cfu/animal/day. When the probiotics are killed or inactivated, the amount of killed or inactivated probiotics or their components should produce a similar beneficial effect as the live microorganisms. Many such probiotics and their benefits are known to skilled artisans, e.g., EP1213970B1, EP1143806B1, US7189390, EP1482811B1, EP1296565B1, and US6929793. In a preferred embodiment, the probiotic is *Enterococcus faecium* SF68 (NCIMB 10415). In one embodiment, the probiotics are encapsulated in a carrier using methods and materials known to skilled artisans.

As stated, the compositions may contain one or more prebiotics, e.g., fructo-oligosaccharides, gluco-oligosaccharides, galacto-oligosaccharides, isomalto-oligosaccharides, xylo-oligosaccharides, soybean oligosaccharides, lactosucrose, lactulose, and isomaltulose. In one embodiment, the prebiotic is chicory root, chicory root extract, inulin, or combinations thereof. Generally, prebiotics are administered in amounts sufficient to positively stimulate the healthy microflora in the gut and cause these "good" bacteria to reproduce. Typical amounts are from about one to about 10 grams per serving or from about 5% to about 40% of the recommended daily dietary fiber for an animal. The probiotics and prebiotics can be made part of the composition by any suitable means. Generally, the agents are mixed with the composition or applied to the surface of the composition, e.g., by sprinkling or spraying. When the agents are part of a kit, the agents can be admixed with other materials or in their own package. Typically, the food composition contains from about 0.1 to about 10% prebiotic, preferably from about 0.3 to about 7%, most preferably from about 0.5 to 5%, on a dry matter basis. The prebiotics can be integrated into the compositions using methods known to skilled artisans, e.g., US5952033.

A skilled artisan can determine the appropriate amount of the compositions, food ingredients, vitamins, minerals, probiotics, prebiotics, antioxidants, or other ingredients to be use to make a particular composition to be administered to a particular animal. Such artisan can consider the animal's species, age, size, weight, health, and the like in determining how best to formulate a particular composition and other ingredients. Other factors that may be considered include the type of composition (e.g., pet food composition versus dietary supplement), the desired dosage of each component, the average consumption of specific types of compositions by different animals (e.g., based on species, body weight, activity/energy demands, and the like), and the manufacturing requirements for the composition.

In a further aspect, the invention provides kits suitable for administering isoflavones to animals. The kits comprise in separate containers in a single package or in separate containers in a virtual package, as appropriate for the kit component, isoflavones and one or more of (1) one or more ingredients suitable for consumption by an animal; (2) instructions for how to combine isoflavones and other kit components to produce a composition useful for regulating sirtuin gene expression, mimicking caloric restriction, preventing and treating Alzheimer's disease, increasing longevity, and retarding aging; (3) instructions for how to use isoflavones for regulating sirtuin gene expression; (4) instructions for how to use isoflavones for mimicking caloric restriction; (5) instructions for how to use isoflavones for preventing and treating Alzheimer's disease; (6) instructions for how to use isoflavones for increasing longevity; (7) instructions for how to use isoflavones for retarding aging; (8) one or more probiotics; 9) one or more inactivated probiotics; (10) one or more components of inactivated probiotics that promote health benefits similar to or the same as the probiotics; (11) one or more prebiotics; (12) a device for preparing or combining the kit components to produce a composition suitable for administration to an animal; and (13) a device for administering the combined or prepared kit components to an animal. In one embodiment, the kit comprises the composition in a sachet.

When the kit comprises a virtual package, the kit is limited to instructions in a virtual environment in combination with one or more physical kit components. The kit contains isoflavones and other components in amounts sufficient for regulating sirtuin gene expression, mimicking caloric restriction, preventing and treating Alzheimer's disease, increasing longevity, and retarding aging. Typically, the isoflavones and the other suitable kit components are admixed just prior to consumption by an animal. The kits may contain the kit components in any of various combinations and/or mixtures. In one embodiment, the kit contains a packet containing isoflavones and a container of food for consumption by an animal. The kit may contain additional items such as a device for mixing isoflavones and ingredients or a device for containing the admixture, e.g., a food bowl. In another embodiment, isoflavones are mixed with additional nutritional supplements such as vitamins and minerals that promote good health in an animal. The components are each provided in separate containers in a single package or in mixtures of various components in different packages. In preferred embodiments, the kits comprise isoflavones and one or more other ingredients suitable for consumption by an animal. Preferably such kits comprise instructions describing how to combine isoflavones with the other ingredients to form a food composition for consumption by the animal, generally by mixing isoflavones with the other ingredients or by applying isoflavones to the other ingredients, e.g., by sprinkling isoflavones on a food composition.

In a further aspect, the invention provides a means for communicating information about or instructions for one or more of (1) using isoflavones for regulating sirtuin gene expression; (2) using isoflavones for mimicking caloric restriction; (3) using isoflavones for preventing and treating Alzheimer's disease; (4) using isoflavones for increasing longevity; (5) using isoflavones for retarding aging; (6) using isoflavones for promoting healthy aging; (7) contact information for consumers to use if they have a question regarding the methods and compositions of the invention; (8) nutritional information about isoflavones; the means comprising one or more of a physical or electronic document, digital storage media, optical storage media, audio presentation, audiovisual display, or visual display containing the information or instructions. The communication means is useful for instructing on the benefits of using the invention and communicating the approved methods for administering isoflavones and food compositions containing isoflavones to an animal. The means comprises one or more of a physical or electronic document, digital storage media, optical storage media, audio presentation, audiovisual display, or visual display containing the information or instructions. Preferably, the means is selected from the group consisting of a displayed website, a visual display kiosk, a brochure, a product label, a package insert, an advertisement, a handout, a public announcement, an audiotape, a videotape, a DVD, a CD-ROM, a computer readable chip, a computer readable card, a computer readable disk, a USB device, a FireWire device, a computer memory, and any combination thereof.

In another aspect, the invention provides methods for manufacturing a food composition comprising isoflavones and one or more other ingredients suitable for consumption by an animal, *e.g.,* one or more of protein, fat, carbohydrate, fiber, vitamins, minerals, probiotics, prebiotics, and the like. The methods comprise admixing one or more ingredients suitable for consumption by an animal with isoflavones. Alternatively, the methods comprise applying isoflavones alone or in conjunction or combination with other ingredients onto the food composition, e.g., as a coating or topping. Isoflavones can be added at any time during the manufacture and/or processing of the food composition. The composition can be made according to any method suitable in the art.

In another aspect, the invention provides a package useful for containing isoflavones or any compositions of the present invention. The package comprises at least one material suitable for containing the isoflavones or any compositions of the present invention and a label affixed to the material containing a word or words, picture, design, acronym, slogan, phrase, or other device, or combination thereof, that indicates that the package contains the isoflavones or any compositions of the present invention with beneficial properties relating to regulating sirtuin gene expression, mimicking caloric restriction, preventing and treating Alzheimer's disease, increasing longevity, retarding aging, and promoting healthy aging. Typically, such device comprises the words "promoting healthy aging," "aging support," "senior support," or an equivalent expression printed on the material. Any package configuration and packaging material suitable for containing isoflavones or any compositions of the present invention are useful in the invention, e.g., a bag, box, bottle, can, pouch, and the like manufactured from paper, plastic, foil, metal, and the like. In preferred embodiments, the package further comprises isoflavones or any compositions of the present invention. In various embodiments, the package further comprises at least one window that permit the package contents to be viewed without opening the package. In some embodiments, the window is a transparent portion of the packaging material. In others, the window is a missing portion of the packaging material. In a preferred embodiment, the package contains a food composition adapted for a particular animal such as a human, canine, or feline, as appropriate for the label, preferably a companion animal food composition for dogs or cats. In a preferred embodiment, the package is a can or pouch comprising a food composition of the invention.

In another aspect, the invention provides for use of isoflavones to prepare a medicament for one or more of regulating sirtuin gene expression; mimicking caloric restriction; preventing and treating Alzheimer's disease; increasing longevity; retarding aging; promoting healthy aging; improving the quality of life; and promoting the health and wellness in an animal. Generally, medicaments are prepared by admixing a compound or composition, i.e., isoflavones or a composition comprising isoflavones, with excipients, buffers, binders, plasticizers, colorants, diluents, compressing agents, lubricants, flavorants, moistening agents, and other ingredients known to skilled artisans to be useful for producing medicaments and formulating medicaments that are suitable for administration to an animal.

In another aspect, the invention provides methods for regulating sirtuin expression by animals. The methods comprise administering one or more isoflavones to the animals in a therapeutically effective amount for regulating sirtuin expression.

For all the methods of the invention, the isoflavones are administered in a therapeutically effective amount. In preferred embodiments, one or more isoflavones are administered to the animals in amounts of from about 0.1 to about 10 grams of isoflavones, preferably from about 0.5 to about 7.5 grams, more preferably from about 1 to about 5 grams. In various embodiments, the isoflavones are administered to the animals in amounts of from about 0.1 to about 10 grams of isoflavones per day (g/day), preferably from about 0.5 to about 7.5 g/day, more preferably from about 1 to about 5 g/day. In other embodiments, the isoflavones are administered to the animals in amounts of from about 0.001 to about 10 grams of isoflavones per kilogram of body weight (g/kg/bw), preferably from about 0.05 to about 5 g/kg/bw, more preferably from about 0.01 to about 1 g/kg/bw. The isoflavones are administered using any suitable method, preferably orally. In one embodiment, the isoflavones are administered as part of a food composition, e.g., integrated into or applied onto the food composition. When integrated into the food compositions, the isoflavones are typically inherent in the ingredients used to produce the food composition, e.g., isoflavones in soy. In another, the isoflavones are administered as part of a dietary supplement, e.g., in a pill or capsule.

### EXAMPLES

The invention can be further illustrated by the following examples, although it will be understood that these examples are included merely for purposes of illustration and are not intended to limit the scope of the invention unless otherwise specifically indicated.

### Example 1

### Materials and Methods

Treatment: Terminally differentiated canine adipocytes were exposed to 200, 25, and 0 uM Equol for 24 hours. Cells were collected for RNA isolation.

RNA Isolation: Treatment cell samples were vortexed and homogenized using a Quiashredder (Qiagen, Valencia, CA) column according to manufacturer's directions. The homogenized lysate was collected and 1 equal volume of 64% ethanol was added to it. This mixture was then applied to an RNAqueous™ filter cartridge, (Ambion Inc., Austin, TX) 700 uL at a time, and centrifuged for one minute at 10,000 rpm. The cartridge was washed using 700uL wash solution #1 and 500 uL wash solution #2/3 with centrifugation at 10,000 rpm for one minute for each wash. The filter cartridge was dried by centrifugation (10,000 rpm) for one minute. RNA was eluted three times by centrifugation (as above) using 30 uL aliquots of 70-80°C elution solution. The resulting RNA was DNAse-treated and quantitated in a Beckman DU 640B spectrophotometer (Beckman Coulter, Inc., Brea, CA) at 260 nm. Additionally, quantity and quality were assessed using a bioanalyzer (Agilent, Santa Clara, CA) according to manufacture's directions.

Quantitative PCR: All samples were run singularly against each primer/probe set to determine what standard curve(s) should be used. Standard curves were generated using serial dilutions of RNA from experimental samples with the lowest CT (cycle threshold) for a given target. 1-2 samples were re-reverse transcribed and a 1:5 serial dilution were used as the standard curve for a given target. Targets run included sirtuin -1, -2, -3 and -6. Values were normalized to cyclophilin A (PPIA) levels as determined by quantitative PCR. Inductions were calculated from each of the lowest sample's normalized value. Each set was run with n=3. The results are shown in Table 1.

**Table 1**

| Expression of Sirtuin Genes in Canine Adipocytes Treated with 0, 25 or 200 uM Equol | | | |
|---|---|---|---|
| | 0 | 25 | 200 |
| SIRT1 | 1.20 | 1.17 | 1.80 |
| SIRT2 | 1.29 | 1.22 | 2.37 |
| SIRT3 | 11.16 | 10.86 | 17.21 |
| SIRT6 | 1.21 | 1.10 | 1.23 |

| | | | |
|---|---|---|---|
| Values Represent Relative Expression | | | |

Referring to Table 1, the results show that gene expression is increased for SIRT1, SIRT2 and SIRT3.

In the specification, there have been disclosed typical preferred embodiments of the invention. Although specific terms are employed, they are used in a generic and descriptive sense only and not for purposes of limitation. The scope of the invention is set forth in the claims. Obviously many modifications and variations of the invention are possible in light of the above teachings. It is therefore to be understood that within the scope of the appended claims the invention may be practiced otherwise than as specifically described.

## Claims

1. Composition comprising one or more isoflavones in a therapeutipically effective amount for use in one or more of the treatment or prevention of Alzheimer's Disease, the mimicking of caloric restriction, increasing longevity, or in retarding aging in animals, wherein said composition comprises the isoflavone equol or a precursor of equol said precursor optionally being daidzein, daidzin, or formononetin.

2. Composition for use according to claim 1, wherein said isoflavone regulates sirtuin gene expression in an animal.

3. Composition for use according to claim 2 wherein the sirtuin gene expressed is at least one of SIRT1, SIRT2, SIRT3, SIRT4, SIRT5 or SIRT7.

4. Composition for use according to 2 wherein the sirtuin gene expressed is at least one of SIRT2 or SIRT3.

5. Composition for use according to 2 wherein the sirtuin gene expressed is at least one of SIRT4, SIRT5 or SIRT7.

6. Composition for use according to any of the above claims wherein the composition further comprises an isoflavone selected from the group consisting of isoflavones in the form of aglycons, glucosides, acetylglucosides, and malonylglucosides.

7. Composition for use according to according to any of the above claims wherein the composition further comprises an isoflavone selected from the group consisting of biochanin A, daidzein, daidzin, glycitein, formononetin, genistein, irilone, luteone, prunetin, pratensein, and glycitinn.

8. Composition for use according to according to any of the above claims wherein isoflavones are to be administered in amounts of from about 0.001 to about 10 g/kg/day.

9. Composition for use according to according to any of the above claims wherein isoflavones are to be administered to the animal on a regular basis.

10. Composition for use according to according to any of the above claims wherein isoflavones are to be administered to the animal as a dietary supplement or a food composition.

11. Composition for use according to claim 10 wherein the isoflavones are to be administered in a food composition and the isoflavones comprise from about 0.001 to about 40% of the food composition.

12. Composition for use according to claim 10 wherein the food composition is formulated to provide complete and balanced nutrition for the animal.

13. Composition for use according to claim 10 wherein the food composition or dietary supplement further comprises one or more probiotics, inactivated probiotics, and components of inactivated probiotics that promote health benefits similar to or the same as the probiotics.

14. Composition for use according to any of the above claims wherein the animal is a human or a companion animal, said companion animal optionally being a canine or a feline.

15. Composition for use according to any of the above claims, wherein the animal is an aging animal.
